# EUROPÄISCHE PATENTSCHRIFT

(11) **EP 1 771 562 B1**
(45) Veröffentlichungstag und Bekanntmachung des Hinweises auf die Patenterteilung: **21.05.2008**
(21) Anmeldenummer: 05761608.8
(22) Anmeldetag: 15.07.2005
(51) Int. Cl.: C12N 15/10, C07H 21/00

(54) **VERFAHREN ZUR REINIGUNG UND ISOLIERUNG VON NUKLEINSÄUREN UNTER VERWENDUNG KATIONISCHER DETERGENTIEN**
METHOD FOR PURIFYING AND ISOLATING NUCLEIC ACIDS USING CATIONIC DETERGENTS
PROCEDE POUR PURIFIER ET ISOLER DES ACIDES NUCLEIQUES PAR UTILISATION DE DETERGENTS CATIONIQUES

(30) Priorität: 15.07.2004 DE 102004034433
(43) Veröffentlichungstag der Anmeldung: 11.04.2007
(73) Patentinhaber: Qiagen GmbH, 40724 Hilden (DE)
(72) Erfinder: SINGER, Thorsten, 42697 Solingen (DE)
(86) Internationale Anmeldenummer: PCT/EP2005/007736
(87) Internationale Veröffentlichungsnummer: WO 2006/008090

(56) Entgegenhaltungen:
- WO-A-00/34463
- DE-A1- 2 403 594
- US-A- 5 234 809
- US-A1- 2003 229 222

## Beschreibung

Die vorliegende Erfindung betrifft ein Verfahren zur Reinigung und Isolierung von Nukleinsäuren unter Verwendung von kationischen Detergentien der allgemeinen Formel:

Y⁺R₁R₂R₃R₄ X⁻ (I)

worin
Y Stickstoff oder Phosphor,
R₁, R₂, R₃ und R₄ unabhängig voneinander einen unverzweigten oder verzweigten C₁-C₂₀-Alkylrest, C₃-C₆-Alkenylrest, C₃-C₆-Alkinylrest und/oder einen C₆-C₂₀-Arylrest sowie einen C₆-C₂₆-Aralkylrest und
X⁻ ein Anion einer anorganischen oder organischen, ein- oder mehrbasischen Säure
bedeuten können.

Bevorzugt sind Kompositionen, in denen die kationische Verbindungen aus einem Ammoniumsalz besteht, in dem R₁ einen höheren Alkylrest, vorzugsweise mit 12, 14, 16 oder 18 Kohlenstoffatomen, und R₂, R₃ und R₄ jeweils eine Methylgruppe bedeutet.

Bevorzugt sind weiterhin Kompositionen, in denen R₁ eine Aralkylgruppe, vorzugsweise eine Benzylgruppe, R₂ einen höheren Alkylrest, vorzugsweise mit 12, 14 oder 16 Kohlenstoffatomen, und R₃ und R₄ eine Methylgruppe bedeutet.

C₁-C₆-Alkyl steht im allgemeinen für einen verzweigten oder unverzweigten Kohlenwasserstoffrest mit 1 bis 6 Kohlenstoffatom(en), der gegebenenfalls mit einem oder mehreren Halogenatom(en) - vorzugsweise Fluor - substituiert sein kann, die untereinander gleich oder verschieden sein können.

Als Beispiele seien folgende Kohlenwasserstoffreste genannt: Methyl, Ethyl, Propyl, 1-Methylethyl (*iso*-Propyl), Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl undl-Ethyl-2-methyl-propyl.

Höherer Alkylrest steht für einen verzweigten oder unverzweigten C₇-C₂₀-Alkylrest der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt: verzweigtes oder unverzweigtes Heptyl, Octyl, Nonyl, Decyl, Undecyl, Dodecyl, Tetradecyl, Hexadecyl, Oktadecyl und Eicosyl.

C₃-C₆-Alkenyl steht im allgemeinen für einen verzweigten oder unverzweigten Kohlenwasserstoffrest mit 3 bis 6 Kohlenstoffatomen, mit einer oder ggf. mehreren Doppelbindungen, der gegebenenfalls mit einem oder mehreren Halogenatom(en) - vorzugsweise Fluor - substituiert sein kann, die untereinander gleich oder verschieden sein können.

Als Beispiele seien folgende Kohlenwasserstoffreste genannt: 2-Propenyl (Allyl), 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 3-Methyl-4-pentenyl, 4-Methyl-4-pentenyl, 1,1-Dimethyl-2-butenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-Butenyl, 2,3-Dimethyl-3-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl.

C₃-C₆-Alkinyl steht im allgemeinen für einen verzweigten oder unverzweigten Kohlenwasserstoffrest mit 3 bis 6 Kohlenstoffatomen, mit einer oder ggf. mehreren Dreifachbindungen, der gegebenenfalls mit einem oder mehreren Halogenatom(en) - vorzugsweise Fluor - substituiert sein kann, die untereinander gleich oder verschieden sein können.

Als Beispiele seien folgende Kohlenwasserstoffreste genannt: 2-Propinyl (Propargyl), 2-Butinyl, 3-Butinyl, 1-Methyl-2-propinyl, 2-Methyl-2-propinyl, 2-Pentinyl, 3-pentinyl, 4-Pentinyl, 1-Methyl-2-butinyl, 2-Methyl-2-butinyl, 3-Methyl-2-butinyl, 1-Methyl-3-butinyl, 2-Methyl-3-butinyl, 3-Methyl-3-butinyl, 1,1-Dimethyl-2-propinyl, 1,2-Dimethyl-2-propinyt, 1-Ethyl-2-propinyl, 2-Hexinyl, 3-Hexinyl, 4-Hexinyl, 5-Hexinyl, 1-Methyl-2-pentinyl, 2-Methyl-2-pentinyl, 3-Methyl-2-pentinyl, 4-Methyl-2-pentinyl, 1-Methyl-3-pentinyl, 2-Methyl-3-pentinyl, 3-Methyl-3-pentinyl, 4-Methyl-3-pentinyl, 1-Methyl-4-pentinyl, 3-Methyl-4-pentinyl, 4-Methyl-4-pentinyl, 1,1-Dimethyl-2-butiriyl, 1,1-Dimethyl-2-butinyl, 1,1-Dimethyl-3-butinyl, 1,2-Dimethyl-2-butinyl, 1,2-Dimethyl-3-butinyl, 1,3-Dimethyl-2-butinyl, 1,3-Dimethyl-3-butinyl, 2,2-Dimethyl-3-butinyl, 2,3-Dimethyl-2-butinyl, 2,3-Dimethyl-3-butinyl, 1-Ethyl-2-butinyl, 1-Ethyl-3-butinyl, 2-Ethyl-1-butinyl, 2-Ethyl-2-butinyl, 2-Ethyl-3-butinyl, 1,1,2-Trimethyl-2-propinyl, 1-Ethyl-1-methyl-2-propinyl und 1-Ethyl-2-methyl-2-propinyl.

Aryl steht - sofern nicht anders definiert - für einen aromatischen ein- oder mehrkernigen Rest mit 4 bis 22 C-Atomen, der ggf. ein oder zwei Heteroatome enthalten kann. Als Beispiele seien genannt: Phenyl, Naphthyl, Anthracyl bzw. Pyrol, Furan, Thiophen, Pyridin, Pyridazin, Pyrimidin oder Pyrazin, und die ggf. mit einem oder mehreren Halogenatom(en) - vorzugsweise Fluor - oder durch eine Alkylgruppe unabhängig voneinander ein- oder mehrfach substituiert sein können.

Aralkyl bedeutet einen ein oder mehrkernigen Arylrest im, Sinne der vorstehenden Definition, der über eine C₁-C₆-Alkylen-, C₃-C₆-Alkenylen- oder eine C₃-C₆-Alkinylenbrücke, für welche die Definition der C₁-C₆-Alkyl-, C₃-C₆-Akenyl- und C₃-C₆-Alkinylgruppen entsprechend gelten, an die kationische Partialstruktur gebunden ist. Im Sinne der vorliegenden Erfindung wird die Benzylgruppe bevorzugt.

Als Anionen werden Bromid, Chlorid, Phosphat, Sulfat, Formiat, Acetat, Propionat, Oxalat oder Succinat bevorzugt.

Verfahren zur Isolierung von Nukleinsäuren sind aus dem Stand der Technik wohlbekannt. Demgemäß erfolgt, nach einer der im Stand der Technik etabliertesten Methoden, die Isolierung von DNA aus biologischen Ausgangsmaterialien - wie z. B. Zellen und Geweben - dadurch, dass die Nukleinsäure enthaltenden Ausgangsmaterialien unter stark denaturierenden und reduzierenden Bedingung (teilweise auch unter Verwendung von proteinabbauenden Enzymen) aufgeschlossen und die so freigesetzten Nukleinsäuren auf dem Wege der Dialyse oder mittels einer Ethanolpräzipitation aus der wässrigen Phase isoliert werden [J. Sambrock, E.F. Fritsch und T. Maniatis, 1989, Cold Spring Harbor, "Molecular Cloning"].

Der wesentliche Nachteil dieser Verfahrensweise ist darin zu sehen, dass sich die Isolierung von Nukleinsäuren aus Zellen und insbesondere aus Geweben als sehr zeitaufwändig erweist und häufig mehr als zwei Tage in Anspruch nehmen kann. Daneben macht diese Verfahrensweise einen erheblichen apparativen Aufwand erforderlich und beinhaltet den Einsatz von hautreizenden bzw. gesundheitsgefährdenden Stoffen wie z. B. Phenol oder Chloroform.

Angesichts dieser Umstände wurden im Stand der Technik frühzeitig alternative Verfahren entwickelt, die es ermöglichen sollten, die oben geschilderten Nachteile bei der Extraktion von Nukleinsäuren zu umgehen.

So beschreibt die US 2003/0229222 A1 ein Verfahren, bei dem gleichzeitig eine Extraktion und eine Aufreinigung von Nukleinsäuren dadurch erfolgt, dass die Nukleinsäure enthaltenden Ausgangsmaterialien zusammen mit einer Mischung aus feinen Glaspartikeln, Chelatisierungsmitteln und quaternären Ammoiniumverbindungen durch starkes Mischen aufgeschlossen werden und durch gleichzeitige Anwesenheit von organischen Lösungsmittel wie z. B. einer Mischung aus Chloroform und Alkoholen aufgereinigt werden.

Ein weiteres Verfahren zur Isolierung einzelner Makropolyanionen wie z. B. Nukleinsäuren aus einem biologischen Material wird in der DE 24 03 594 A1 beschrieben. Das dortige Verfahren erfolgt über eine fraktionierte Fällung verschiedener Nukleinsäuren unter Verwendung eines Salzgemischs der Nukleinsäuren mit quartären, einen langkettigen Alkylrest enthaltenden Ammoniumkationen und eines starken Elektrolyten, wobei die Konzentration des starken Elektrolyten vorzugsweise bei gleichbleibender Konzentration des Ammoniumkations verringert wird und dabei nacheinander die Salze der jeweiligen Nukleinsäuren ausfallen.

Weiters Verfahren basieren auf einer von Vogelstein und Gillespie (Proc. Natl. Acad. Sci, USA, 1979, 76, 615-619) entwickelten und erstmals beschriebenen

Methode zur präparativen und analytischen Reinigung von DNA-Fragmenten aus Agarosegelen. Die Methode kombiniert die Auflösung der die zu isolierende DNA-Bande enthaltenden Agarose in einer gesättigten Lösung eines chaotropen Salzes (NaJ) mit einer Bindung der DNA an Glaspartikel. Die an die Glaspartikel fixierte DNA wird anschließend mit einer Waschlösung (20 mM Tris HCl [pH 7,2], 200 mM NaCl; 2 mM EDTA; 50% v/v Ethanol) gewaschen und danach von den Trägerpartikeln desorbiert.

Diese Methodik erfuhr bis heute eine Reihe von Modifikationen und wird zum gegenwärtigen Zeitpunkt für unterschiedliche Verfahren der Extraktion und Reinigung von Nukleinsäuren unterschiedlicher Provenienz angewendet (Marko, M.A., Chipperfield, R. und Birnboim, H.G., Anal. Biochem., 121, (1982) 382-387).

Zur Durchführung derartiger Nukleinsäureextraktionen sind demgemäß zahlreiche Reagenzien-Zusammenstellungen (sog. Kits) kommerziell erhältlich.

Diese fast ausschließlich kommerziell verfügbaren Kits basieren auf dem hinlänglich bekannten Prinzip der Bindung von Nukleinsäure an mineralische Träger unter Anwesenheit von Lösungen unterschiedlicher chaotroper Salze, und verwenden als Trägermaterialien Suspensionen fein gemahlener Glaspulver (z. B. Glasmilk, BIO 101, La Jolla, CA), Diatomeenerden (Fa. Sigma) oder auch Silicagele (Europäische Patentanmeldung 616 639).

Ein für eine Vielzahl von unterschiedlichen Anwendungen prinzipiell praktikables Verfahren zur Isolierung von Nukleinsäuren ist in Boom et al. [EP 389 063 A1] offenbart worden. In dieser Europäischen Patentanmeldung wird ein Verfahren zur Isolierung von Nukleinsäuren aus nukleinsäurehaltigen Ausgangsmaterialien durch die Inkubation des Ausgangsmaterials mit einem chaotropen Puffer aus einer DNAbindenden festen Phase beschrieben. Die chaotropen Puffer realisieren sowohl die Lyse des Ausgangsmaterials als auch die Bindung der Nukleinsäuren an die feste Phase. Das Verfahren ist geeignet, um Nukleinsäuren auch aus kleinen Probenmengen zu isolieren und findet speziell im Bereich der Isolierung viraler Nukleinsäuren seine praktische Anwendung.

Probleme, die durch eine ggf. schwierige Lyse des Ausgangsmaterials erwachsen, können zwar durch eine Reihe von kommerziell verfügbaren Produkten für die Nukleinsäureisolierung (speziell für die Isolierung genomischer DNA aus komplexen Ausgangsmaterialien) gelöst werden, bergen aber den großen Nachteil in sich, dass es sich hierbei nicht mehr um ein klassisches "Single Tube-Verfahren" handelt, dass das o. a. Verfahren gemäß der Offenbarung von Boom et al. kennzeichnet, da die Lyse des Ausgangsmaterials in einem gebräuchlichen Puffer unter Einbeziehung eines proteolytischen Enzyms erfolgt. Die für die nachfolgende Bindung der Nukleinsäuren, an z. B. Zentrifugationsmembranen, notwendigen chaotropen Ionen müssen nach erfolgter Lyse dem Lyseansatz extra zugegeben werden. Sie können aber in keinem Fall Bestandteil des Lysepuffers sein, da die proteinzerstörende Funktion chaotroper Salze bekannt ist und natürlich sofort das für eine effiziente Lyse notwendige proteolytische Enzym ebenfalls zerstören würde.

Trotz einer Reihe von Nachteilen haben sich die Methoden der Nukleinsäureisolierung unter der Verwendung chaotroper Salze weltweit durchgesetzt, und werden mittels kommerziell verfügbarer Produkte millionenfach eingesetzt. Diese Systeme sind extrem einfach in ihrer Durchführung und verfahren immer nach folgendem Prinzip:
- Lyse des Ausgangsmaterials, nachfolgende Bindung der Nukleinsäure an die feste Phase einer Glas- oder Silicamembran, welche sich in einem Zentrifugationssäulchen an einer Trägersuspension befindet;
- Waschen der gebundenen Nukleinsäuren und
- Elution der Nukleinsäuren mit einem Puffer geringer Ionenstärke;

Alle diese Systeme beruhen auf der Bindung der Nukleinsäuren an die jeweiligen Trägeroberflächen in Anwesenheit chaotroper Salze, d. h. mindestens eine Pufferlösung enthält als Hauptkomponente ein chaotropes Salz. Dies betrifft u. U. schon den Lysepuffer oder - bei Systemen unter Einbeziehung proteolytischer Enzyme - einen notwendigen Bindungspuffer, welcher nach der erfolgten Lyse des Ausgangsmaterials zugegeben wird.

Die Basis für die Auswahl geeigneter chaotroper Salze bilden die Reihen von Hofrneister zur Aussalzung von negativ geladenen, neutralen oder basischen Eiweißlösungen. Die chaotropen Salze sind dadurch charakterisiert, Proteine zu denaturieren, die Löslichkeit unpolarer Substanzen in Wasser zu erhöhen sowie hydrophobe Wechselwirkungen zu zerstören. Gerade diese Eigenschaften bewirken nach dem Stand der Technik auch mit Puffersystemen chaotroper Salze die übergeordnete Struktur des wässrigen Milieus zu zerstören, um so die Bindung der Nukleinsäuren an ausgewählte feste Phasen zu vermitteln. Die bekanntesten Vertreter zur Nukleinsäureisolierung sind, Natriumperchlorat, Natriumjodid, Kaliumiodid, Guanidinium-*iso*-thiocyanat und Guanidinium-Hydrochlorid. Sie sind jedoch zum einen kostenintensiv und zum anderen teilweise toxisch oder ätzend.

Das physico-chemische Prinzip der Bindung von Nukleinsäuren an mineralische Träger in Anwesenheit chaotroper Salze gilt in der Fachwelt als erklärt. Die Bindung der Nukleinsäuren an die Oberflächen der mineralischen Träger bestehe in der Störung übergeordneter Strukturen des wässerigen Milieus, durch welche die Nukleinsäuren an die Oberfläche von mineralischen Materialien, insbesondere von Glas- bzw. Silicapartikeln adsorbieren. Zur Störung der übergeordneten Strukturen des wässerigen Milieus ist immer die Anwesenheit chaotroper Ionen erforderlich. Bei hohen Konzentrationen der chaotropen Salze verläuft die Reaktion fast quantitativ. Aufgrund dieser beschriebenen physiko-chemischen Erkenntnisse wird deshalb im Stand der Technik davon ausgegangen, dass alle kommerziell verfügbaren Systeme zur Isolierung von Nukleinsäuren, Pufferkompositionen mit hohen Ionenstärken chaotroper Salze für die Bindung von Nukleinsäuren an eine nukleinsäurenbindende feste Phase enthalten müssen.

Daneben sind aus dem Stand der Technik auch entsprechende Verfahren bekannt, die ohne die Anwendung von chaotropen Substanzen oder ohne den Einsatz von Phenol auskommen, womit die oben geschilderten Nachteile vermieden werden können. So offenbart die Internationale Patentanmeldung WO 00/034463 derartige Verfahren zur Isolierung von Nukleinsäuren aus komplexen Ausgangsmaterialien. Bei diesen Verfahren kommt ein Lyse/Bindungspuffersystem zum Einsatz, das mindestens eine antichaotrope Salzkomponente aufweist und mit einer sog. alkoholischen Bindechemie arbeitet (Invisorb^{®} Plasmid Kit der Fa. Invitek, Berlin).

Der Einsatz von Alkoholen weist allerdings ebenfalls einige Nachteile auf, zu denen insbesondere die folgenden gehören:
- Es müssen ganz bestimmte Volumenverhältnisse von Probelysat zu Alkohol für die Plasmidisolierung peinlich genau eingehalten werden, um die Kontamination des zu isolierenden Plasmids - insbesondere mit RNA - zu vermeiden.
- Sowohl die chaotrope als auch die alkoholische Bindechemie resultieren in DNA-Isolaten, die stark mit Endotoxinen kontaminiert sind. Zusätzliche Waschschritte sind dabei auf die Verwendung stark alkoholhaltiger Puffer eingeschränkt, um die Bindung an die feste Phase - meist eine Membran - aufrechterhalten zu können. Selektive Waschschritte, die den Einsatz anderer Substanzen erforderlich machen, können daher kaum zu diesem Zweck eingesetzt werden.
- Der Gehalt an Endotoxinen in der isolierten DNA kann zu Problemen bei dem weiteren Einsatz der so isolierten DNA - beispielsweise in pharmazeutischen Anwendungen - führen.

Die Aufgabe der vorliegenden Erfindung besteht demzufolge darin, zunächst die oben geschilderten Nachteile des Standes der Technik zu überwinden.

Gelöst wird diese Aufgabe durch den Einsatz von kationischen Detergentien der allgemeinen Formel (I), um die Nukleinsäuren an eine feste Matrix zu binden. Geeignete Matrizes sind aus dem Stand der Technik bekannt - wie beispielsweise Cellulose. Erfindungsgemäß bevorzugt wird allerdings eine Silica- bzw. Glasfaser-Matrix.

In den Grundzügen lässt sich die prinzipielle Abfolge des erfindungsgemäßen Verfahrens, am Beispiel einer Plasmidpräparation wie folgt beschreiben:

Zunächst werden die Bakterien aus dem sie enthaltenden Medium isoliert, wonach sie praktischerweise in Form eines Pellets vorliegen. Im nachfolgenden Schritt wird das Pellet unter Verwendung eines Resuspendierungspuffers resuspendiert. Derartige Resuspendierungspuffer sind aus dem Stand der Technik bekannt (beispielsweise: 50 mM Tris-C1, pH 8,0, 10 mM EDTA, mit RNase A).

Die so erhaltene. Suspension wird mit einem Lysepuffer versetzt (die hier einsetzbaren Lysepuffer sind dem Fachmann ebenfalls aus dem Stand der Technik bekannt), wie zum Beispiel 200 mM NaOH, 1 % SDS. Nach der Lyse wird der Ansatz - je nach Ausführungsform - mittels eines Neutralisationspuffers neutralisiert. Auch die hier einsetzbaren Neutralisationspuffer sind dem Fachmann aus dem Stand der Technik in großer Zahl geläufig. Sie umfassen beispielsweise Puffer wie eine wässrige 3 M Kaliumacetat-Lösung mit einem pH-Wert von 5.

Das resultierende Reaktionsgemisch wird gegebenenfalls filtriert. Geeignete Filter sind aus dem Stand der Technik wohlbekannt und kommerziell erhältlich (wie z. B. QIAfilter^{®} Midi der Firma OIAGEN, D-40724 Hilden). Danach wird das Lysat mit einer oder mehreren Verbindungen der allgemeinen Formel (I), vorzugsweise in wässriger Lösung, versetzt, und nach dem Vermischen auf eine Silicasäule, vorzugsweise eine sog. Spinsäule, gegeben. (Je nach Durchführungsprotokoll kann für eine "mildere Lyse" die Verbindung der allgemeinen Formel (I) bereits dem Lysepuffer zugegeben werden. / s. a. Teil 4)

Falls das innerhalb der Spin-Säule zur Verfügung stehende Volumen zur Aufnahme der gesamten Menge nicht ausreicht, kann auf die Spinsäule ein Extender in Form eines Trichters aufgesetzt werden, der die Aufnahme der gesamten Flüssigkeitsmenge ermöglicht.

Das Reaktionsgemisch wird praktischerweise unter Anlegen eines Vakuums, oder durch zentrifugieren durch die Säule transportiert bzw. gesogen, wobei die gewünschte Nukleinsäure, hier die Plasmid-DNA, an die Oberfläche, vorzugsweise an eine Silica-Oberfläche, bindet.

Danach wird die Säule mit einem Waschpuffer ggf. mehrfach gewaschen. Derartige Waschpuffer sind aus dem Stand der Technik ebenfalls bekannt und umfassen Puffer, wie z. B. "Puffer PE" (Firma QIAGEN, Hilden).

Eventuell vorhandene Reste an Waschpuffer können erforderlichenfalls mittels Zentrifugation vom Säulenmaterial entfernt werden.

Abschließend wird die gebundene Nukleinsäure mit einem ebenfalls aus dem Stand der Technik bekannten Elutionspuffer von der Säule eluiert.

Demgemäß betrifft die vorliegende Erfindung ein Verfahren Isolierung und Reinigung von Nukleinsäuren, das sich allgemein durch folgende Schritte beschreiben lässt:
- lysieren einer Nukleinsäure enthaltende biologische Probe;
- gegebenenfalls neutralisieren des aus der Lyse resultierenden Ansatzes;
- versetzen des so erhaltenen Reaktionsgemisches mit einer oder mehreren Verbindungen der allgemeinen Formel (1) oder deren wässeriger Lösung, und in Kontakt bringen des Reaktionsgemisches mit einer auf Silica basierenden Matrix;
- waschen der Nukleinsäuren;
- isolieren der Nukleinsäuren.

Des Weiteren betrifft die vorliegende Erfindung das oben beschriebene Verfahren, in dem als Lysepuffer eine wässerige Lösung enthaltend 200 mM NaOH und 1 Gew.-%N SDS eingesetzt wird.

Des Weiteren betrifft die vorliegende Erfindung das oben beschriebene Verfahren in dem als Neutralisationspuffer eine wässrige 2 - 3 M Kaliumacetat-Lösung eingesetzt wird.

Des Weiteren betrifft die vorliegende Erfindung das oben beschriebene Verfahren in dem als Waschpuffer vorzugsweise eine wässrige 300 bis 1000 mM, besonders bevorzugt eine 400 bis 800 mM, ganz besonders bevorzugt eine 500 bis 600 mM Kochsalz-Lösung eingesetzt wird.

Des Weiteren betrifft die vorliegende Erfindung das oben beschriebene Verfahren in dem als Waschpuffer eine bei einem pH-Wert von 7.5 puffernde, 70 - 90 %ige wässrige Lösung von Ethanol eingesetzt wird. Entsprechende Puffer sind aus dem Stand der Technik bekannt und umfassen beispielsweise u. a. Tris/HCl, MOPS oder ähnliche Puffergemische.

Daneben betrifft die vorliegende Erfindung die Verwendung von Verbindungen der allgemeinen Formel (I) zur reversiblen Bindung von Nukleinsäuren an eine mineralische Matrix, wobei die Matrix aus einem porösen und/oder nicht-porösen Träger auf der Basis von Metalloxiden oder Metallmischoxiden aufgebaut sein kann, und vorzugsweise aus einer Silizium-Sauerstoffverbindung, besonders bevorzugt Siliziumdioxid (Silica), bzw. aus einem Silicat oder einem Glas, Silicagel bzw. einem Zeolith bestehen kann.

Daneben kann die mineralische Matrix aus Aluminiumoxid, Titandioxid oder Zirkondioxid bestehen bzw. es können Mischungen der genannten Metalloxide eingesetzt werden.

Insbesondere betrifft die vorliegende Erfindung die Verwendung von Verbindungen der allgemeinen Formel (I) zur Bindung von Nukleinsäuren, wobei diese in Form von einzel- und/oder doppelsträngiger DNA und/oder RNA sowie einzelner Nukleotide und/oder Ribonukleotide vorliegen können, und es sich im Falle von DNA namentlich um genomische DNA, Plasmid-DNA, und/oder plastidäre DNA, und im Falle von RNA um mRNA, tRNA, rRNA und/oder sn-RNA handelt.

Schlussendlich betrifft die vorliegende Erfindung ein Kit zum Isolieren und oder Reinigen von Nukleinsäuren, mindestens enthaltend eine Verbindung der allgemeinen Formel (I).

### Beispiele:

Die vorliegende Erfindung wird nunmehr durch die nachfolgenden Beispiele erläutert:
Folgende Abkürzungen werden verwandt:
   - CTAB: Cetyltrimethylammoniumbromid
   Hexadecyltrimethylammoniumbromid
   - TTAB: Tetradecyltrimethylammoniumbromid
   - DoTAB: Dodecyltrimethylammoniumbromid
   - DTAB: Decyltrimethylammoniumbromid
   - OTAB: Octcyltrimethylammoniumbromid
   - GEL: Ausbeute bestimmt über eine densitometrische Auswertung eines Agarosegels
   - min: Minute(n)
   - Puffer PE: handelsüblicher Waschpuffer (Firma QIAGEN, D-40724 Hilden)
   - Puffer EC: handelsüblicher Puffer zum Einsatz in der Zellkultur (Firma QIAGEN, D-40724 Hilden)
   - Puffer RLT: handelsüblicher Lyse-/Bindepuffer (Firma QIAGEN, D-40724 Hilden)
   - Puffer EB: handelsüblicher Elutionspuffer (Firma QIAGEN, D-40724 Hilden)
   - Puffer DP3: handelsüblicher Neutralisationspuffer (Firma QIAGEN, D-40724 Hilden)
   - Puffer S3: handelsüblicher Neutralisationspuffer (Firma QIAGEN, D-40724 Hilden)
   - Puffer CE3: handelsüblicher Extraktionspuffer (Firma QIAGEN, D-40724 Hilden)
   - OD: Ausbeute bestimmt über photometrische Messung bei 260 nm (optische Dichte)
   - RT: Raumtemperatur (ca. 20 - 25 °C)
   - X: Durchschnittswert der Mehrfachbestimmungen

### Teil 1: Reinigung und Isolierung von Nukleinsäuren unter Verwendung von kationischen Detergentien

Die Beispiele 1 bis 4 wurden - sofern nicht anders angegeben - nach dem folgenden Protokoll durchgeführt:
Protokoll (1) für Plasmid-DNA Präparationen aus *E.coli* im "Midi-Maßstab":
   1. Bakterienpellet in 2 ml Resuspendierungspuffer resuspendieren;
   2. Zugabe von 2 ml Lysepuffer - anschließend ca. 3 min lysieren;
   3. Zugabe von 2 ml Neutralisationspuffer, mischen durch invertieren;
   4. sofortiges Überführen in einen QIAfilter^{®} Midi, 3 min bei RT inkubieren und filtrieren;
   5. 10 ml Extender auf die Spin-Säule setzen und auf QIAvac^{®} positionieren;
   6. Zugabe von 2 ml der Detergentienlösung zum Lysat, durchmischen und auf die Säule geben;
   7. durchsaugen der Mischung, Vakuum aufheben;
   8. Extender verwerfen;
   9. Spin-Säule in Collection-Tube setzen;
   10. optionales Waschen der Membran durch Zugabe von 750 µl Salzpuffer, 1 min bei 14 000 Upm zentrifugieren;
   11. waschen der Membran durch Zugabe von 750 µl Puffer PE, 1 min bei 14 000 Upm zentrifugieren;
   12. zum Entfernen von Pufferresten noch einmal 1 min bei 14 000 Upm zentrifugieren;
   13. Spin-Säule auf 1,5 ml Eppendorf-Tube platzieren;
   14. Elution mit 200 µl Puffer EB. Auf die Membran pipettieren, 1 min inkubieren und zentrifugieren (1 min bei 14 000 Upm).

### Beispiel 1: Vergleich der erfindungsgemäßen Bindechemie unter Verwendung von Detergentien gegenüber der sog. "alkoholischen" Bindechemie

Gemäß dem vorliegenden Protokoll (1) wurden 25 ml einer DH5α/pCMVβ Kultur (high-copy Plasmid) mit jeweils 2 ml, 2,5 ml und 4 ml einer Detergentienlösung (4 Gew-% in 0,5 M NaCl) bzw. mit Isopropanol gefällt:

**Tabellen 1 und 2**

| **CTAB-Bindung, Ausbeuten OD₂₆₀ (µg)** | | | | | ***iso*-Propanol-Bindung, Ausbeuten OD₂₆₀ (µg)** | | | |
|---|---|---|---|---|---|---|---|---|
| | **2 ml** | **2,5 ml** | **4 ml** | | | **2 ml** | **2,5 ml** | **4 ml** |
| 1 | 448 | 460 | 502 | | 1 | 331 | 439 | 716 |
| 2 | 453 | 532 | 379 | | 2 | 325 | 381 | 679 |
| **X** | **450** | **491** | **440** | | **X** | **328** | **410** | **698** |

**Tabellen 3 und 4**

| **CTAB-Bindung, Ausbeuten Gel (µg)** | | | | | **CTAB-Bindung, Ausbeuten Gel (µg)** | | | |
|---|---|---|---|---|---|---|---|---|
| | **2 ml** | **2,5 ml** | **4 ml** | | | **2 ml** | **2,5 ml** | **4 ml** |
| 1 | 421 | 456 | 384 | | 1 | 308 | 428 | 354 |
| 2 | 421 | 464 | 357 | | 2 | 374 | 374 | 352 |
| **X** | **421** | **460** | **370** | | **X** | **341** | **401** | **353** |

Wie die obigen Daten belegen, weist das Detergentien-Bindesystem bei gleichen bis höheren Ausbeuten eine größere Stabilität gegenüber einer auf Alkohol basierenden Bindung auf.

### Beispiel 2: Steuerung der Bindung und Selektivität über die Salzkonzentration

In dem vorliegenden Beispiel werden je 25 ml einer DHSα/pBRCMVβ Kultur (low-copy Plasmid) entsprechend dem Protokoll (1) mit CTAB bei verschiedenen Salzkonzentrationen gebunden.

In den folgenden Tabellen 5 und 6 sind jeweils die Mittelwerte einer Doppelbestimmung wiedergegeben:

| **CTAB-Bindung, Ausbeuten OD260 (µg)** | | | | | | |
|---|---|---|---|---|---|---|
| **0,4 M** | **0,5 M** | **0,6 M** | **0,7 M** | **0,8 M** | **0,9 M** | **1,0 M** |
| 40 | 26 | 21 | 12 | 14 | 11 | 220 |

| **CTAB-Bindung, Ausbeuten Gel (µg)** | | | | | | |
|---|---|---|---|---|---|---|
| **0,4 M** | **0,5 M** | **0,6 M** | **0,7 M** | **0,8 M** | **0,9 M** | **1,0 M** |
| 9 | 8 | 11 | 9 | 14 | 8 | 0 |

Die experimentellen Ergebnisse zeigen deutlich, dass sich durch die Wahl einer geeigneten Salzkonzentration optimale Bedingungen für die selektive Bindung einer gewünschten Nukleinsäure einstellen lassen. Außerdem liefern die identischen Werte der photo- und der densitometrischen Bestimmung bei einer Konzentration von 0.8 M NaCl einen Beleg dafür, dass sich in der jeweiligen Präparation ausschließlich die gewünschte Plasmid-DNA befindet.

### Beispiel 3: Verwendung salzhaltiger Waschpuffer zur Entfernung von RNA Kontaminationen

Bei Betrachtung der kationischen Detergentien als "lösliche Anionenaustauscher" sollte es möglich sein, mittels eines Stufengradienten, unerwünschte Nukleinsäuren nach der Bindung auf der Membran abzulösen, und so eine zusätzliche Selektivität der Bindung zu erhalten.

Im dargestellten Beispiel wurden je 50 ml einer DH5α/pBRCMVβ Kultur (low-copy Plasmid) entsprechend dem Protokoll (1) mit 1% CTAB in 0,8 M NaCl an die Membran gebunden. Dann wurde die gebundene DNA mit Waschpuffern verschiedener Kochsalzkonzentrationen gewaschen (optionaler Schritt 10 im Protokoll (1)):
a) kein optionaler Waschschritt
b) 300 mM NaCl
c) 600 mM NaCl
d) 800 mM NaCl

**Tabellen 7 und 8**

| **CTAB-Bindung, Ausbeuten OD₂₆₀ (µg)** | | | | | | **CTAB-Bindung, Ausbeuten Gel (µg)** | | | | |
|---|---|---|---|---|---|---|---|---|---|---|
| | **a)** | **b)** | **c)** | **d)** | | | **a)** | **b)** | **c)** | **d)** |
| 1 | 122 | 60 | 23 | 1,7 | | 1 | 47 | 28 | 19 | 0,2 |
| 2 | 97 | 76 | 43 | 1,4 | | 2 | 47 | 41 | 48 | 0,4 |
| **X** | **110** | **68** | **33** | **1,5** | | **X** | **47** | **35** | **34** | **0,3** |

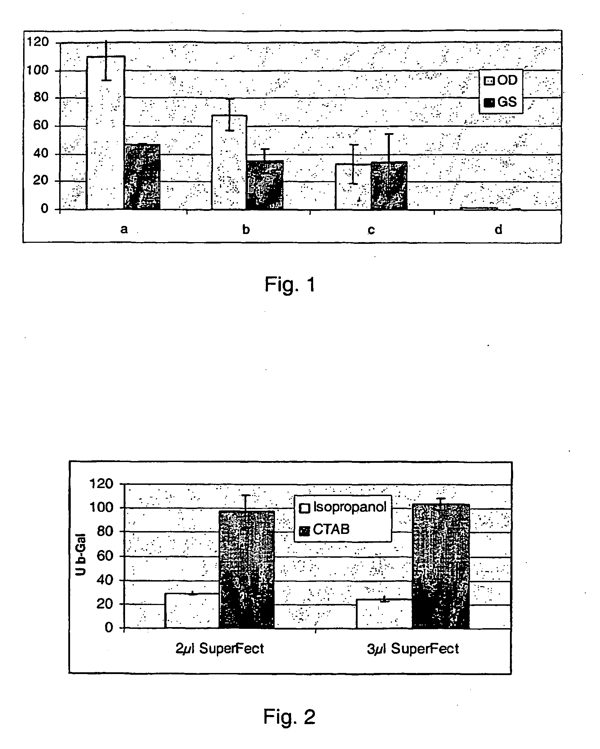

Die in den oben stehenden Tabellen aufgeführten experimentellen Daten werden in Fig.1 graphisch wiedergegeben. Im Ergebnis zeigt sich, dass sich die Detergenz-Nukleinsäure-Komplexe auf der Membran wie Nukleinsäuren verhalten, die an einen Anionenaustauscher gebunden werden. Daher lassen sich Verunreinigungen bei geeigneter Salzkonzentration mit einem Waschpuffer selektiv entfernen.

Ferner konnte gezeigt werden, dass mit allen oben angegebenen Detergentien mit einer Kettenlänge von mehr als acht Kohlenstoffatomen, Plasmid DNA identischer Reinheit (OD/Gel-Werte praktisch identisch) an eine Silica-Membran gebunden werden kann. Die Ausbeuten hängen dabei vom verwendeten Detergenz und der dabei verwendeten Salzkonzentrationen (Ionenstärken) der Lösung ab.

Hierbei konnte gezeigt werden, dass eine Bindung auch bei niedrigen Salzkonzentrationen eintritt, bei denen keine Inhibierung der Präzipitation der DNA stattfindet.

### Beispiel 4: Qualität der isolierten DNA:

Zur Beurteilung der Qualität der isolierten DNA wurden Vergleiche zwischen alkoholischer Bindechemie und der Bindung mit einem kationischen Detergenz durchgeführt. Die Ergebnisse sind in den nachfolgenden Tabellen 9 und 10 dargestellt:

### a) Untersuchung auf Endotoxin-Kontamination (EU/µg DNA):

| high-copy Plasmid: | | |
|---|---|---|
| | ***iso*-Propanol** | **CTAB** |
| 1 | 1241. | 3 |
| 2 | 1466 | 11 |
| **X** | **1354** | **7** |

| low-copy Plasmid (ohne zusätzlichen Waschschritt): | | |
|---|---|---|
| | ***iso*-Propanol I** | **CTAB** |
| 1 | 2395 | 15 |
| 2 | 2536 | 19 |
| **X** | **2466** | **17** |

Während der Endotoxingehalt bei Anwendung einer alkoholischen Bindechemie im üblichen Bereich für DNA Präparationen mittels Silica-Technologie liegt, liegen die Verunreinigungen bei den CTAB-Präparationen in einem Bereich, der normalerweise nur unter Verwendung von Anionenaustauschersäulen erreicht wird.

### b) Ergebnisse der Transfektion in endotoxinsensitiven Zellen (Huh7):

Transfektionen gelten als kritische Anwendungen, für welche die verwandte Plasmid-DNA möglichst frei von Verunreinigungen (besonders Endotoxinen) sein muss. Die Zellen wurden entsprechend dem SuperFect-Protokoll (Firma QIAGEN, D-40724 Hilden) transfiziert. Als Referenz (100%) diente das mittels einer Anionenaustauschersäule (Ultrapure100 Firma QIAGEN, D-40724 Hilden) isolierte Plasmid.

Die erzielten Ergebnisse sind in der Fig. 2 wiedergegeben.

Die Ergebnisse der Transfektionen zeigten für die mit CTAB isolierte DNA-Werte von ca. 100%, d. h. identisch zu den Präparationen mit einer Anionenaustauschersäule (Ultrapure 100).

Dagegen ergaben die Präparationen mit *iso*-Propanol-Bindung, im Einklang mit den hohen Endotoxinwerten (s. o.) signifikant schlechtere Transfektionsergebnisse.

### Teil 2: Verzicht auf vorhergehende Klärung des Zell-Lysats

Die Beispiele 5 bis 8 wurden - sofern nicht anders angegeben - nach dem folgenden Protokoll durchgeführt:
Protokoll (2) für eine Schnellpräparation von Plasmid DNA aus *E.coli* im "MiniMaßstab" (für 1,5 ml Bakterienkultur):
   1. Bakterienpellet in 150 µl Resuspendierungspuffer resuspendieren;
   2. Zugabe von 150µl Lysepuffer. Ca. 3 min lysieren;
   3. Zugabe von 150µl Neutralisationspuffer, mischen durch invertieren;
   4. Zugabe von 300µl der Detergenzienlösung zum Lysat, gut durchmischen und auf die Säule geben;
   5. zentrifugieren (1 min 14 000 Upm);
   6. waschen durch Zugabe von 750 µl Puffer PE, 1 min bei 14 000 Upm zentrifugieren.
   7. Zum Entfernen von Pufferresten noch einmal 1 min bei 14 000 Upm zentrifugieren.
   8. Spin-Säule auf 1,5 ml Eppendorf-Tube platzieren;
   9. Elution mit 100 µl Puffer EB. Auf die Membran pipettieren, 1 min stehen lassen und zentrifugieren (1 min bei 14 000 Upm).

### Beispiel 5: Optimierung der Bindung bei Verzicht auf die Lysatklärung

in dem vorliegenden Beispiel wurden je 1,5 ml einer DH5α/pBRCMVβ Kultur (high-copy) entsprechend dem Protokoll (2) mit CTAB bei verschiedenen Salzkonzentrationen gebunden.
Angegeben sind jeweils die Mittelwerte einer Doppelbestimmung.

Basierend auf den Ergebnissen mit Lysatklärung wurde die notwendige Salzkonzentration in der Detergenzlösung optimiert:
Eingesetzt wurden je 300 µl einer 1 % CTAB-Lösung mit den angegebenen Salzkonzentrationen.

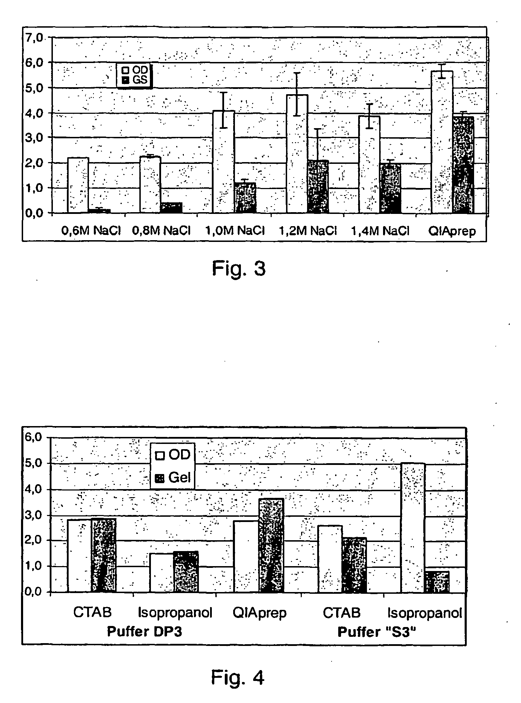

Wie Fig. 3 zu entnehmen ist, wurden die besten Ergebnisse mit 1,2 bzw. 1,4 M NaCl erhalten. Zu beachten ist dabei, dass mit Lysatklärung bei diesen Salzkonzentrationen keine Bindung mehr stattfindet. Dem gegenüber führten 0,8 M NaCl, dem ermittelten Optimum bei Lysatklärung (vgl. Beispiel 2), hier zu deutlich schlechteren Ergebnissen.

### Beispiel 6: Präparation im Spinformat

In dem vorliegenden Beispiel wurden je 1,5 ml einer DH5α/pCMVβ Kultur (high-copy) entsprechend dem Protokoll (2) mit 1 % CTAB in 1,2 M NaCl bzw. Isopropanol gefällt.

Als Neutralisierungspuffer wurde jeweils der Puffer DP3 (3 M Ammoniumacetat, pH 5,5) sowie der Puffer S3 (2M Kaliumacetat, pH 5,5) verwendet. Eine QIAprep Präparation der QIAGEN GmbH diente als zusätzlicher Vergleich.

Wie Fig. 4 zeigt, wird mit dem kationischen Detergenz CTAB eine doppelt so hohe Ausbeute erreicht, wie bei der Verwendung von Isopropanol. Die Präparationen mit dem Puffer S3 zeigten für die kationische Bindung nur leicht geringere Ausbeuten und nach wie vor eine gute Übereinstimmung zwischen OD und Gel. Demgegenüber stand eine sehr hohe Überquantifzierung in der OD bei der Verwendung von isopropanol.

### Beispiel 7: Präparation im 96 well Format, automatisiert

In dem vorliegenden Beispiel wurde eine Schüttelkolbenanzucht von DH5α/pCMVβ Kultur (high-copy) als 1,5 ml Aliquots in eine 96 well Platte aufgeteilt.
Je eine 96 well Platte wurde anschließend auf einem Analysenroboter (z. B. dem BR8000 / QIAGEN GmbH) mit CTAB bzw. Isopropanol zur DNA Bindung aufgearbeitet.

Für die Isolierung mit Isopropanol wurde das aktuellste DP96 Protokoll gewählt (je 100 µl der Lysepuffer, 180µl Isopropanol; so genanntes "low-volume-protocol"). Für die Isolierung mit CTAB wurde eine Protokollversion gewählt, die möglichst nahe an dem bisher verwendeten Spin-Protokoll liegt (je 150µl Lysepuffer, 300µl CTAB-Lösung).

Bei allen 96 wells wurde die Ausbeute per OD₂₆₀ bestimmt (s. Tabellen 11 und 12). Zusätzlich wurde bei je sieben wells noch eine densitometrische Analyse eines Agarosegels zur Ausbeutebestimmung durchgeführt.

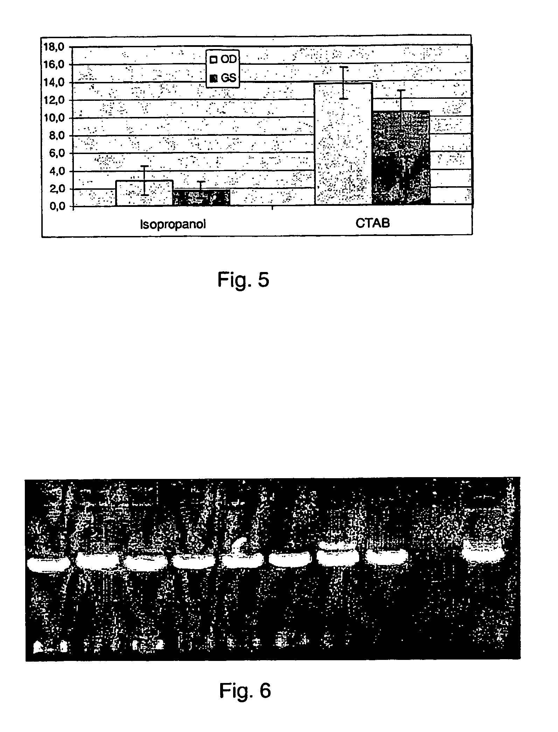

Fig. 5 zeigt den Vergleich OD und Gel von sieben willkürlich ausgewählten wells. In der gewählten Reihe wird der Vorteil der neuen Bindechemie bei der Ausbeute sehr deutlich sichtbar. Die Übereinstimmung zwischen OD und Gel ist in beiden Fällen gleich gut.

Bei den gemittelten Werten aus den nachstehenden Tabellen 11 und 12 über alle 96 wells fällt der Unterschied weniger deutlich aus, als bei den ausgewählten sieben Proben, bleibt jedoch ca. 2-3 mal höher.

### CTAB:

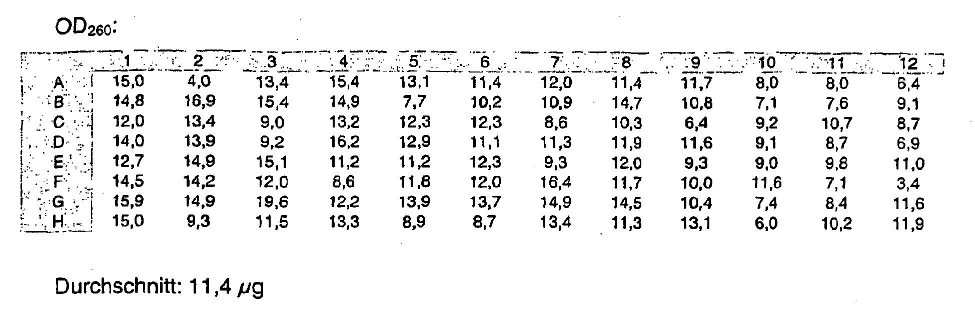

### Isopropanol:

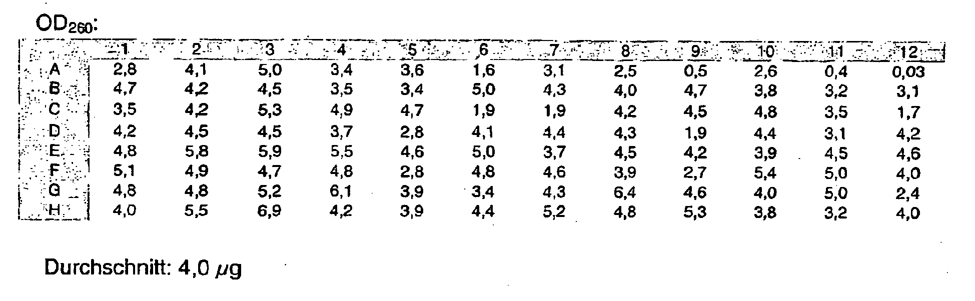

### Beispiel 7: Qualität der erhaltenen DNA

Zur Bestimmung der Qualität der isolierten DNA wurden verschiedene Applikationen durchgeführt:

### a) Restriktionsanalyse

Die Proben der in Beispiel 7 isolierten Plasmid DNA wurden mit einem salzempfindlichen Restriktionsenzym verdaut. Als Kontrolle wurde dasselbe Plasmid verwendet, welches jedoch vorab mit einer Anionenaustauschersäule (z. B. einer UP100 / QIAGEN GmbH) isoliert wurde.

Fig. 6 zeigt deutlich, dass alle verdauten Proben die erwartete zusätzliche Bande bei 0,7 kb aufweisen. Ein Unterschied zur Kontrolle war nicht zu erkennen.

### b) Sequenzierbarkeit

Wie der nachstehenden Tabelle 13 zu entnehmen ist, zeigen alle eingesetzten DNA Proben eine sehr gute Leselänge ("Readlength") von im Mittel ca. 800 bp. Auch die Signalstärken der einzelnen Basen, sowie der frühe Beginn der Lesbarkeit zeigen, dass die Qualität der isolierten Plasmid-DNA für die Sequenzierung sehr gut geeignet ist.

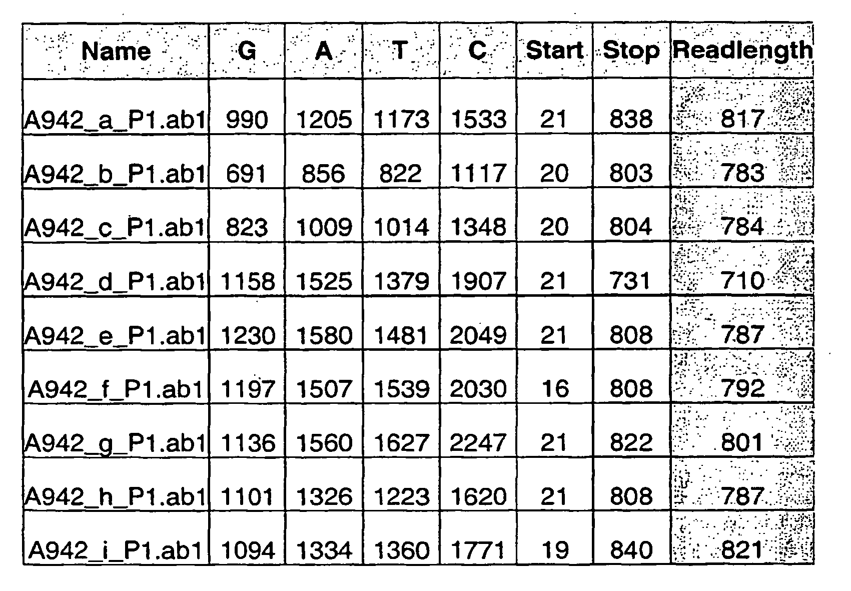

### c) Bestimmung des Endotoxingehalts:

Der Endotoxingehalt der Proben der Reihe 4 wurde mittels des LAL Kinetic Kit (Fa. Cambrex) bestimmt.

### Endotoxinunits/µg eingesetzte DNA:

| Reihe 4 | A | B | C | D | E | F | G | H | Durchschnitt |
|---|---|---|---|---|---|---|---|---|---|
| | 2442 | 2420 | 778 | 2230 | 1242 | 1329 | 1968 | 1833 | **1780** |

Die Messung ergab Endotoxinkontaminationen, die in der Größenordnung einer herkömmlichen Silika-Präparation mit chaotroper Bindechemie und Lysatklärung liegen (vgl. Beispiel 4).

Demgegenüber liegen die Werte bei der alkoholischen Bindechemie ohne Lysatklärung um ca. einen Faktor 10 höher.

### Fazit:

Wie die Beispiele 5 bis 7 zeigen, erlaubt die neue Bindechemie in Analogie zur alkoholischen Bindechemie (s. a. WO 03/040364) ebenfalls einen Verzicht auf die Lysatklärung, was v. a. im Hinblick auf Hochdurchsatzanwendungen bzw. einer Automatisierung von großem Vorteil ist.

Wie Beispiel 6 zeigt, kann das automatisierte Protokoll (2) sehr leicht von bestehenden Systemen übernommen werden. Im Vergleich mit der alkoholischen Bindechemie liefert die kationische Bindechemie Plasmid DNA, die - bei deutlich höheren Ausbeuten - in ihrer Qualität gleichwertig bis besser ist.

### Teil 3: Nachreinigung von DNA

Nachfolgend werden einige Beispiele zur Reinigung von DNA in beliebiger Größe aufgeführt. Es wurde dabei u. a. auf die Abtrennung von freien Nukleotiden oder Primern nach enzymatischen Reaktionen, sowie die selektive Isolierung von Primern neben größeren DNA Molekülen abgezielt.

Die Beispiele 9 bis 11 wurden - sofern nicht anders angegeben - nach dem folgenden Protokoll (3) durchgeführt, welches die Abfolge der Nachreinigung mit kationischen Detergentien zur Bindung an eine Silika-Membran im Minimaßstab zeigt.

Protokoll (3) zur selektiven Bindung von DNA in Abhängigkeit der Größe
1. Probe mit Detergenzlösung versetzen;
2. gut durchmischen und auf die Säule geben;
3. zentrifugieren (1 min 14 000 Upm);
4. waschen durch Zugabe von 750 µl Puffer PE, 1 min bei 14 000 Upm zentrifugieren.
5. Zum Entfernen von Pufferresten noch einmal 1 min bei 14 000 Upm zentrifugieren.
6. Spin-Säule auf 1,5 ml Eppendorf-Tube platzieren;
7. Elution mit 100µl Puffer EB. Auf die Membran pipettieren, 1 min stehen lassen und zentrifugieren (1 min bei 14 000 Upm).

Die aufgeführten Beispiele 9 bis 11 demonstrieren drei unterschiedliche Parameter, die zur Unterscheidung der Bindung von verschiedenen DNA-Molekülen ausgenutzt werden können:
- Menge des eingesetzten Detergenz
- pH-Wert
- im Bindeansatz enthaltene Ionen (Kationen)

Diese drei Parameter sollten genügend Kombinationsmöglichkeiten bieten, um auf einfache Weise jede gewünschte Selektivität erreichen zu können.

### Beispiel 9: Selektive Bindung eine 100bp Fragments

Je 2µg 100bp-Fragment bzw. 20mer wurden in 50µl Puffer EB gelöst, mit verschiedenen Verdünnungen einer TTAB Lösung versetzt (je 300µl) und weiter nach dem oben beschriebenen Protokoll (3) behandelt.

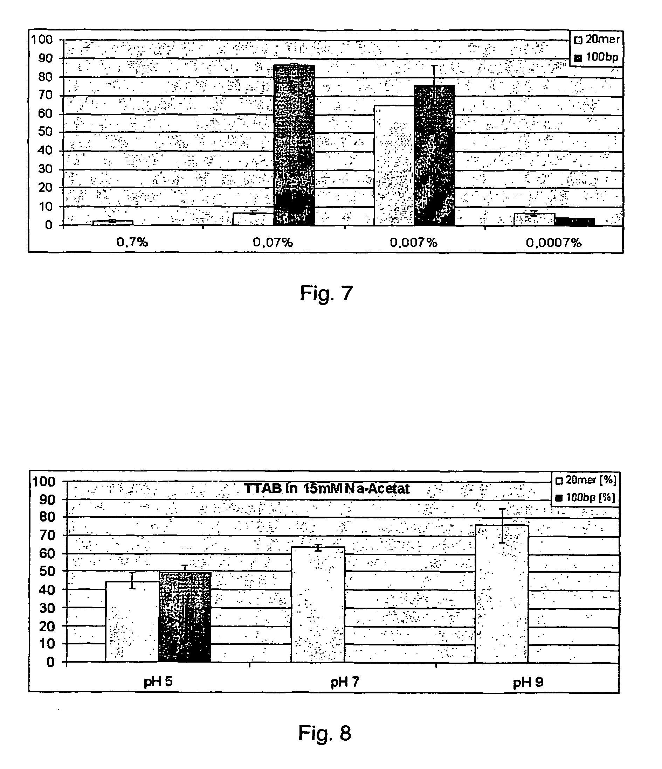

Das in Fig. 7 dargestellte Ergebnis zeigt, dass allein durch die Menge an eingesetztem Detergenz (hier: 0,07%) eine fast quantitative Abreicherung des 20mers erreicht werden kann.

### Beispiel 10: Selektive Bindung eines 20mer Oligonukleotids durch Wahl eines geeigneten pH-Wertes

Es wurden je 1µg eines 20mers bzw. 100bp Fragments in 100µl Puffer EB (10 mM Tris/HCl, pH 8,5) gelöst und mit 300µl einer 0,05%igen acetatgepufferten TTAB Lösung versetzt (15 mM Essigsäure/Na-Acetat, pH Wert 5; 7 und 9). Die Proben wurden weiter nach dem oben beschriebenen Protokoll (3) behandelt.

Das in Fig. 8 dargestellte Ergebnis zeigt eine Wiederfindung und damit Bindung von sowohl dem 20mer als auch dem 100bp Fragment bei pH5. Im Gegensatz dazu wurde bei höheren pH-Werten lediglich das 20mer gebunden. Dies zeigt, dass mittels der erfindungsgemäßen Bindechemie eine gewünschte Trennung von DNA-Fragmenten einzig durch Wahl des geeigneten pH-Wertes erreicht werden kann.

### Beispiel 11: Selektive Bindung oder Nichtbindung eines 100bp Fragments durch Wahl eines geeigneten Kations

Es wurden je 2µg eines 20mers bzw. 100bp Fragments bzw. pUC21 Plasmid in 50µl Puffer EB (10 mM Tris/HCl, pH 8,5) gelöst und mit 50µl der entsprechenden Acetatlösung (s. nachfolgende Auflistung a bis d), mit einem pH-Wert von etwa 5,5 versetzt. Dazu wurden 50µl einer 4% CTAB-Lösung in 500 mM NaCl gegeben und weiter nach dem oben beschriebenen Protokoll (3) behandelt.
a) 2 M Kalium-Acetat
b) 1 M Magnesium-Acetat
c) 1 M Calcium-Acetat
d) 1 M Mangan(II)-Acetat

Während das 20mer in jedem Fall abgetrennt wurde, blieb das Plasmid pUC21 in jedem Fall gebunden. Das 100bp Fragment konnte je nach verwendetem Kation gebunden oder nicht gebunden werden.

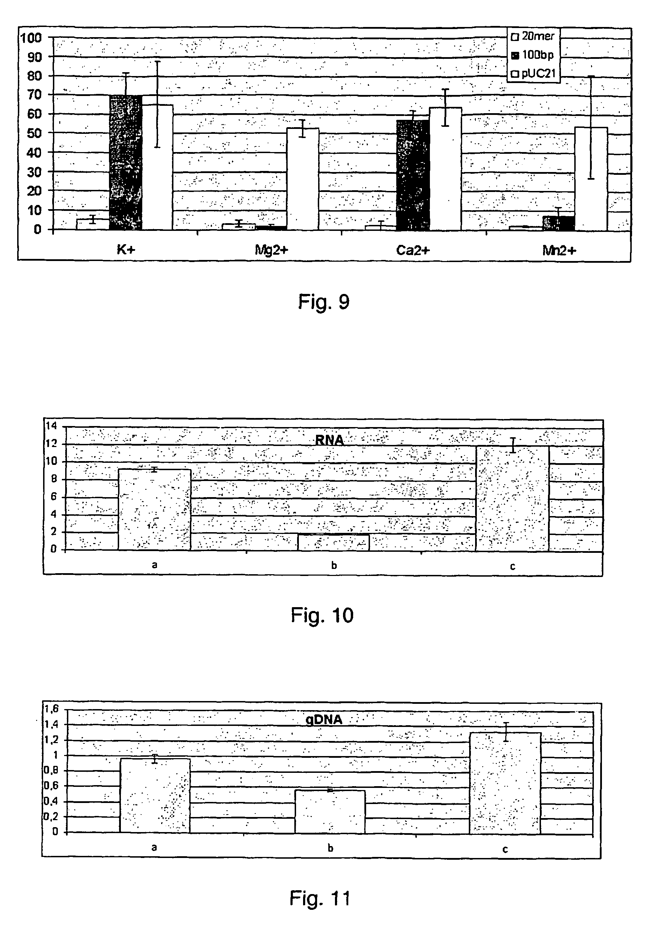

So zeigt die Fig. 9, dass allein durch die Wahl des verwendeten Kations bereits eine gewisse Selektivität für die gewünschten DNA Moleküle erreicht werden kann.

### Teil 4: Isolierung von RNA (incl. DNA sowie der nativen Proteine)

Im Folgenden wird gezeigt, wie die neue Bindechemie auch zur Isolierung von RNA aus biologischem Material eingesetzt werden kann. Dabei findet die Lyse durch einen üblichen milden Lysepuffer statt, wie er z.B. zur Isolierung von Proteinen seit langem eingesetzt wird. Dieser Puffer enthält aber erfindungsgemäß das kationische Detergenz, das einerseits die RNA stabilisiert und andererseits die Bindung an die Silika-Membran vermittelt.

Bei diesem Verfahren werden im initialen Bindeschritt alle Nukleinsäuren gebunden und diese dann selektiv mit bereits bekannten Puffern nacheinander eluiert.

Im Gegensatz zu den bekannten, üblichen Verfahren, bei denen die RNA durch Inaktivierung der RNasen bei hohen Konzentrationen chaotroper Salze stabilisiert wird, wird bei der Bindung an kationische Detergentien die RNA vor dem Angriff geschützt. D. h. die in der Probe vorhandenen Proteine behalten ihre biologische Funktion und können in nativer Form aus dem Durchbruch der Silica-Spinsäule weiter aufgearbeitet werden.

Dies ergibt ganz wesentliche Vorteile gegenüber den bisher bekannten Systemen, bei denen die Probe geteilt werden musste, wobei die eine Hälfte für die Isolierung nativer Proteine bei gleichzeitiger RNA Degradation, und die zweite Hälfte zur Isolierung von Nukleinsäuren bei gleichzeitiger Denaturierung der Proteine verwendet wird, was neben dem Nachteil jeweils die Hälfte der Proben verwerfen zu müssen, bei einer Aufteilung von inhomogenem Probenmaterial sehr leicht zu verfälschten Ergebnissen führen kann.
Mit dem erfindungsgemäßen Verfahren ist es zudem erstmals möglich aus ein und demselben Lysat funktionelle Studien auf RNA- und Proteinebene durchzuführen.

Vorteilhafterweise kann dabei noch zusätzlich die genomische DNA isoliert werden, was bei Analysen, bei denen die Beobachtungen auf ihre Ursachen im genetischen Hintergrund (Mutationen) geprüft werden, von großer Bedeutung sein kann.

Die nachstehenden Beispiele 12 und 13 wurden - sofern nicht anders angegeben - nach dem folgenden Protokoll durchgeführt:

### Protokoll (4) zur Isolierung von RNA, gDNA und nativen Proteinen aus einer Probe (Protokoll für ein 12 well Zellkulturformat)

1. Medium abnehmen und Zellen mit 1 ml Puffer EC waschen;
2. Platte auf Eis 5 min kühlen;
3. Zellen mit 400µl Lysepuffer (1,6 Vol Extraction Buffer CE 3 + 1 Vol 8% TTAB in Wasser) versetzen und 5 min bei Raumtemperatur stehen lassen. (Als Lysepuffer kann jeder allgemein gebräuchliche Puffer für die Isolierung nativer Proteine als Basis dienen, der dann mit einer geeigneten Menge eines kationischen Detergenz versetzt wird.)
4. Zellen abschaben und Lysat durch 3x auf und ab pipettieren homogenisieren;
5. und das Lysat durch eine handelsübliche Spin Säule ( z. B. eine RNeasy-Spin Säule / QIAGEN GmbH) zentrifugieren.
6. Im Durchbruch befindet sich die **Protein Fraktion**, die nachfolgend separat analysiert wird.
7. Die mit der gewonnenen Nukleinsäure beladene Spinsäule wird mit 350µl eines handelsüblichen chaotropen Puffers (z. B. RLT-Puffer / QIAGEN GmbH) gespült;
8. der Durchbruch wird aufgefangen und in Schritt 10 weiterbehandelt.
9. Die Spinsäule wird nachfolgend mit PE Puffer gewaschen und die auf der Säule verbliebene **gDNA** mit EB Puffer eluiert.
10. Den die RNA enthaltende RLT-Durchbruch mit 250µl 100% Ethanol versetzen;
11. durch eine neue RNeasy Spinsäule zentrifugieren
12. und die gewünschte **RNA**, beispielsweise mit dem "RNeasy Mini Protokoll for Isolation of Total RNA from Animal Cells" / Schritt 4, S. 32 / aus dem RNeasy Mini Handbook, QIAGEN GmbH / Stand Juni 2001, isolieren.

### Beispiel 12: Isolierung von RNA und DNA

Hela S3 Zellen wurden im 12 well Zellkulturformat angezogen und je vier wells mit zwei verschiedenen gängigen Proteinlysepuffern (s. Auflistung a und b) sowie CTAB als Detergenz lysiert. Als Referenz dienten entsprechende RNeasy Präparationen c) (QIAGEN GmbH).
a) β-G Galaktosidase -Lysepuffer (s.u.) mit 1%CTAB in 100mM NaCl (Endkonzentration 188 mM)
b) Cell Extraktion Buffer CE3 mit 1% CTAB in 300mM NaCl (Endkonzentration 804 mM)
c) RNeasy Mini (Fa. QIAGEN)

| | |
|---|---|
| β-Galaktosidase-Lysepuffer: | 10 mM Tris, pH 7,4 |
| | 1 mM EDTA |
| | 100 mM NaCl |
| | 5 mM MgCl₂ |
| | 1% NP40 |

Nachfolgend wurde entsprechend dem oben aufgeführten Protokoll (4) die RNA und gDNA isoliert.

Die in den Figuren 10 und 11 dargestellten Ergebnisse zeigen deutlich, insbesondere im Vergleich der Ansätze a) und c), dass mit dem erfindungsgemäßen Verfahren in Kombination mit den bereits bekannten Puffern eine gleichzeitige Isolierung von RNA und DNA möglich ist. Dabei sind Ausbeute und Qualität den etablierten Systemen vergleichbar.

Eine im Laufe der Entwicklung durchgeführte Optimierung der NaCl Konzentration für den Lysepuffer CE3 auf finale 375 mM führte nachfolgend ebenfalls zu vergleichbaren Ergebnissen.

### Beispiel 13: Qualität der isolierten Proteine

HeLa S3 Zellen wurden im 12 well Zellkulturformat angezogen und mit einem das β-Galaktosidase-Gen tragenden Plasmid transfiziert. Nach 48 Stunden wurden die Zellen gemäß dem oben beschriebenen Protokoll (4) lysiert, wobei je vier wells mit zwei verschiedenen gängigen Lysepuffern (s. Auflistung a und b) versetzt wurden.

Als Referenz (s. Auflistung c) diente die Lyse mit einem Standard-Lysepuffer, wie er bei β-Galaktosidase Aktivitätsmessungen verwendet wird.
a) Lysepuffer mit 3% TTAB
b) Lysepuffer mit 3% TTAB, anschließend durch RNeasy Spinsäule zentrifugiert
c) Standard β-Galakiosidase Lysepuffer (Zusammensetzung s. Beispiel 12)

Die Proteinfraktion wurde anschließend zur Ausfällung des TTABs mit einem Volumen einer 3% SDS Lösung versetzt. Die Aktivität des Enzyms wurde vor und nach der SDS-Fällung gemessen.

Wie anhand der gelben Färbung der Reaktionslösungen mit β-Galaktosidase in den wells (ohne Abbildung) demonstriert werden konnte, sind die erhaltenen Proteine nach wie vor biologisch aktiv. Dies ist bei im Stand der Technik hinlänglich bekannten Verfahren, die mit hoch molaren, chaotropen und damit stark denaturierenden Puffern arbeiten, die eine RNase-Inaktivierung zum Ziel haben, nicht möglich.

## Patentansprüche

1. Verfahren zur Isolierung und Reinigung von Nukleinsäuren aus einem Lysat **dadurch gekennzeichnet, dass** man
a) eine Nukleinsäure enthaltende biologische Probe mit einem Lysepuffer lysiert;
b) den aus der Lyse resultierenden Ansatz gegebenenfalls mit einem Neutralisationspuffer neutralisiert;
c) das so erhaltene Reaktionsgemisch mit einer oder mehreren Verbindungen der allgemeinen Formel (I)
Y⁺R₁R₂R₃R₄ X⁻ (I)
worin
Y Stickstoff oder Phosphor
R₁, R₂, R₃ und R₄ unabhängig voneinander einen unverzweigten oder verzweigten C₁-C₂₀-Alkylrest, C₃-C₆-Alkenylrest, C₃-C₆-Alkinylrest und/oder einen C₆-C₂₀-Arylrest sowie einen C₆-C₂₆-Aralkylrest und
X⁻ ein Anion einer anorganischen oder organischen, ein- oder mehrbasischen Säure
bedeuten können, oder deren wässeriger Lösung versetzt, und mit einer mineralischen Matrix, bevorzugt mit einer auf Silica basierenden Matrix, in Kontakt bringt,
d) die Nukleinsäuren mit einem Waschpuffer wäscht;
e) die Nukleinsäuren isoliert.

2. Verfahren nach Anspruch 1, **dadurch gekennzeichnet, dass** R₁ einen höheren Alkylrest vorzugsweise mit 12, 14, 16 oder 18 Kohlenstoffatomen bedeutet, R₂, R₃ und R₄ jeweils eine Methylgruppe bedeuten und Y Stickstoff bedeutet.

3. Verfahren nach Anspruch 1, **dadurch gekennzeichnet, dass** R₁ einen Aralkylrest, vorzugsweise eine Benzylgruppe, R₂ einen höheren Alkylrest, vorzugsweise mit 12, 14 oder 16 Kohlenstoffatomen, bedeutet und R₃ und R₄ jeweils eine Methylgruppe bedeuten.

4. Verfahren nach einem der Ansprüche 1 bis 3, **dadurch gekennzeichnet, dass** das Gegenion X Bromid, Chlorid, Phosphat, Sulfat, Formiat, Acetat, Propionat, Oxalat oder Succinat ist.

5. Verfahren nach einem der Ansprüche 1 bis 4, **dadurch gekennzeichnet, dass** als Lysepuffer eine wässerige Lösung enthaltend 200 mM NaOH und 1 Gew-%/V SDS eingesetzt wird.

6. Verfahren nach einem der Ansprüche 1 bis 5, **dadurch gekennzeichnet, dass** als Neutralisationspuffer eine 2 - 3 M wässrige Kaliumacetat-Lösung eingesetzt wird.

7. Verfahren nach einem der Ansprüche 1 bis 6, **dadurch gekennzeichnet, dass** als Waschpuffer vorzugsweise eine wässrige 300 bis 1000 mM, besonders bevorzugt eine 400 bis 800 mM, ganz besonders bevorzugt eine 500 bis 600 mM Kochsalz-Lösung und/oder als Waschpuffer eine bei einem pH-Wert von 7.5 puffernde, 70 - 90 %ige wässrige Ethanol-Lösung, vorzugsweise enthaltend Tris/HCl, eingesetzt wird.

8. Verwendung von Verbindungen der allgemeinen Formel (I)
Y⁺R₁R₂R₃R₄ X⁻ (I)
worin
Y Stickstoff oder Phosphor
R₁, R₂, R₃ und R₄ unabhängig voneinander einen unverzweigten oder verzweigten C₁-C₂₀-Alkylrest, C₃-C₆-Alkenylrest, C₃-C₆-Alkinylrest und/oder einen C₆-C₂₀-Arylrest sowie einen C₆-C₂₆-Aralkylrest und
X⁻ ein Anion einer anorganischen oder organischen, ein- oder mehrbasischen Säure
darstellen zur Bindung von Nukleinsäuren an eine mineralische Matrix.

9. Verwendung nach Anspruch 8, **dadurch gekennzeichnet, dass** R₁ einen höheren Alkylrest vorzugsweise mit 12, 14, 16 oder 18 Kohlenstoffatomen bedeutet, R₂, R₃ und R₄ jeweils eine Methylgruppe bedeuten und Y Stickstoff bedeutet.

10. Verwendung nach Anspruch 8, **dadurch gekennzeichnet, dass** R₁ eine Aralkylgruppe, vorzugsweise eine Benzylgruppe, R₂ einen höheren Alkylrest, vorzugsweise mit 12, 14 oder 16 Kohlenstoffatomen, bedeutet und R₃ und R₄ jeweils eine Methylgruppe bedeuten.

11. Verwendung nach einem der Ansprüche 8 bis 10, **dadurch gekennzeichnet, dass** das Gegenion X Bromid, Chlorid, Phosphat, Sulfat, Formiat, Acetat, Propionat, Oxalat oder Succinat ist.

12. Verwendung nach einem der Ansprüche 8 bis 11, **dadurch gekennzeichnet, dass** die mineralische Matrix aus einem porösen und/oder nicht-porösen Träger auf der Basis von Metalloxiden oder Metallmischoxiden aufgebaut ist.

13. Verwendung nach Anspruch 12, **dadurch gekennzeichnet, dass** die mineralische Matrix aus einer Silizium-Sauerstoff-Verbindung, bevorzugt Siliziumdioxid, bzw. aus einem Silikat besteht.

14. Verwendung nach Anspruch 12, **dadurch gekennzeichnet, dass** die mineralische Matrix aus Glas, Silicagel bzw. einem Zeolith besteht.

15. Verwendung nach Anspruch 12, **dadurch gekennzeichnet, dass** die mineralische Matrix aus Aluminiumoxid, Titandioxid und/oder Zirkondioxid besteht.

16. Verwendung nach einem der Ansprüche 13 bis 15, **dadurch gekennzeichnet, dass** Mischungen der genannten Metalloxide eingesetzt werden.

17. Verwendung nach einem der Ansprüche 8 bis 16 zur Bindung von Nukleinsäuren, insbesondere in Form von einzel- und/oder doppelsträngiger DNA und/oder RNA sowie einzelner Nukleotide und/oder Ribonukleotide.

18. Verwendung nach Anspruch 17, **dadurch gekennzeichnet, dass** es sich bei der DNA um genomische DNA, Plasmid-DNA und/oder plastidäre DNA handelt.

19. Verwendung nach Anspruch 17, **dadurch gekennzeichnet, dass** es sich bei der RNA um mRNA, tRNA, rRNA und/oder sn-RNA handelt.

## Claims

1. Method for the isolation and purification of nucleic acids from a lysate,
**characterised in that**
a) a biological sample comprising a nucleic acid is lysed with a lysis buffer;
b) the assay resulting from the lysis is optionally neutralised with a neutralization buffer;
c) the reaction mixture thus obtained is treated with one or more compounds of the general formula (I)
Y⁺R₁R₂R₃R₄ X⁻ (I)
wherein
Y can stand for nitrogen or phosphorus
R₁, R₂, R₃ and R₄ independently of one another can represent a linear or branched C₁-C₂₀ alkyl group, C₃-C₆ alkenyl group, C₃-C₆ alkynyl group and/or a C₆-C₂₀ aryl group and a C₆-C₂₆ aralkyl group and
X⁻ can be an anion of an inorganic or organic mono- or polyprotic acid,
or is treated with an aqueous solution thereof, and is brought into contact with a mineral matrix, preferably with a silica-based matrix,
d) the nucleic acids are washed with a wash buffer;
e) the nucleic acids are isolated.

2. Method according to claim 1, **characterised in that** R₁ stands for a higher alkyl group, preferably with 12, 14, 16 or 18 carbon atoms, R₂, R₃ and R₄ in each case stand for a methyl group and Y stands for nitrogen.

3. Method according to claim 1, **characterised in that** R₁ stands for an aralkyl group, preferably a benzyl group, R₂ for a higher alkyl group, preferably with 12, 14 or 16 carbon atoms, and R₃ and R₄ in each case stand for a methyl group.

4. Method according to any one of claims 1 to 3, **characterised in that** the counterion X is bromide, chloride, phosphate, sulphate, formate, acetate, propionate, oxalate or succinate.

5. Method according to any one of claims 1 to 4, **characterised in that** an aqueous solution comprising 200 mM NaOH and 1 wt%/V SDS is used as lysis buffer.

6. Method according to any one of claims 1 to 5, **characterised in that** a 2 - 3 M aqueous potassium acetate solution is used as neutralisation buffer.

7. Method according to any one of claims 1 to 6, **characterised in that** an aqueous 300 to 1000 mM, particularly preferred a 400 to 800 mM, most particularly preferred a 500 to 600 mM sodium chloride solution and/or a 70 - 90 % aqueous ethanol solution buffered to a pH value of 7.5, preferably containing Tris/HCl, is used as wash buffer.

8. Use of compounds of the general formula (I)
Y⁺R₁R₂R₃R₄ X⁻ (I)
wherein
Y can stand for nitrogen or phosphorus
R₁, R₂, R₃ and R₄ independently of one another represent a linear or branched C₁-C₂₀ alkyl group, C₃-C₆ alkenyl group, C₃-C₆ alkynyl group and/or a C₆-C₂₀ aryl group as well as a C₆-C₂₆ aralkyl group and
X⁻ is an anion of an inorganic or organic mono- or polyprotic acid
for the binding of nucleic acids to a mineral matrix.

9. Use according to claim 8, **characterised in that** R₁ stands for a higher alkyl group preferably with 12, 14, 16 or 18 carbon atoms, R₂, R₃ and R₄ in each case stand for a methyl group and Y stands for nitrogen.

10. Use according to claim 8, **characterised in that** R₁ stands for an aralkyl group, preferably a benzyl group, R₂ stands for a higher alkyl group, preferably with 12, 14 or 16 carbon atoms and R₃ and R₄ in each case stand for a methyl group.

11. Use according to any one of claims 8 to 10, **characterised in that** the counterion X is bromide, chloride, phosphate, sulphate, formate, acetate, propionate, oxalate or succinate.

12. Use according to any one of claims 8 to 11, **characterised in that** the mineral matrix is constructed from a porous and/or non-porous metal oxide-based or metal mixed oxide-based support.

13. Use according to claim 12, **characterised in that** the mineral matrix is comprising of a silicon-oxygen compound, preferably silicon dioxide, or a silicate.

14. Use according to claim 12, **characterised in that** the mineral matrix is comprising of glass, silica gel or a zeolite.

15. Use according to claim 12, **characterised in that** the mineral matrix is comprising of aluminium oxide, titanium dioxide and/or zirconium dioxide.

16. Use according to any one of claims 13 to 15, **characterised in that** mixtures of the named metal oxides are used.

17. Use according to any one of claims 8 to 16 for the binding of nucleic acids, particularly in the form of single stranded and/or double stranded DNA and/or RNA as well as of single nucleotides and/or ribonucleotides.

18. Use according to claim 17, **characterised in that** the DNA is genomic DNA, plasmid DNA and/or plastidal DNA.

19. Use according to claim 17, **characterised in that** the RNA is mRNA, tRNA, rRNA and/or sn-RNA.

## Revendications

1. Procédé d'isolation et de purification d'acides nucléiques à partir d'un lysat, **caractérisé en ce que**
a) un échantillon biologique qui contient de l'acide nucléique est lysé avec un tampon de lyse,
b) l'ensemble obtenu après la lyse est éventuellement neutralisé avec un tampon de neutralisation,
c) le mélange de réaction ainsi obtenu est mélangé avec un ou plusieurs composés de formule générale (I):
Y⁺R₁R₂R₃R₄ X⁻ (I)
dans laquelle
Y peut représenter l'azote ou le phosphore,
R₁, R₂, R₃ et R₄ peuvent représenter indépendamment l'un de l'autre un groupe alkyle en C₁ à C₂₀, un groupe alcényle en C₃ à C₆, un groupe alcényle en C₃ à C₆ et/ou un groupe aryle en C₆ à C₂₀ et un groupe aralkyle en C₆ à C₂₆, ramifiés ou non ramifiés, et
X⁻ peut représenter un anion d'un acide mono- ou polyfonctionnel, minéral ou organique,
ou une solution aqueuse de ces composés, et est mis en contact avec une matrice minérale, de préférence une matrice à base de silice,
d) les acides nucléiques sont lavés avec un tampon de lavage et
e) les acides nucléiques sont isolés.

2. Procédé selon la revendication 1, **caractérisé en ce que** R₁ représente un groupe alkyle supérieur qui compte de préférence 12, 14, 16 ou 18 atomes de carbone, R₂, R₃ et R₄ représentent chacun un groupe méthyle et Y représente l'azote.

3. Procédé selon la revendication 1, **caractérisé en ce que** R₁ représente un groupe aralkyle, de préférence un groupe benzyle, R₂ représente un groupe alkyle supérieur qui compte de préférence 12, 14 ou 16 atomes de carbone et R₃ et R₄ représentent chacun un groupe méthyle.

4. Procédé selon l'une des revendications 1 à 3, **caractérisé en ce que** le contre-anion X⁻ représente un bromure, chlorure, phosphate, sulfate, formiate, acétate, propionate, oxalate ou succinate.

5. Procédé selon l'une des revendications 1 à 4, **caractérisé en ce que** comme tampon de lyse, on utilise une solution aqueuse qui contient NaOH 200 mM et 1 % en volume de SDS.

6. Procédé selon l'une des revendications 1 à 5, **caractérisé en ce que** comme tampon de neutralisation, on utilise une solution aqueuse d'acétate de potassium 2 à 3 M.

7. Procédé selon l'une des revendications 1 à 6, **caractérisé en ce que** comme tampon de lavage, on utilise de préférence une solution aqueuse de sel de cuisine 300 à 1 000 mM, de façon particulièrement préférable 400 à 800 mM et de façon tout particulièrement préférable 500 à 600 mM et/ou **en ce que** l'on utilise comme tampon de lavage une solution aqueuse d'éthanol à 70-90 % qui tamponne le pH à une valeur de 7,5 et qui contient de préférence du Tris/HCl.

8. Utilisation de composés de formule générale (I)
Y⁺R₁R₂R₃R₄ X⁻ (I)
dans laquelle
Y représente l'azote ou le phosphore,
R₁, R₂, R₃ et R₄ représentent indépendamment l'un de l'autre un groupe alkyle en C₁ à C₂₀, un groupe alcényle en C₃ à C₆, un groupe alcinyle en C₃ à C₆ et/ou un groupe aryle en C₆ à C₂₀ ou un groupe aralkyle en C₆ à C₂₆, ramifiés ou non ramifiés, et
X⁻ peut représenter un anion d'un acide mono- ou polyfonctionnel, minéral ou organique,
pour lier les acides nucléiques à une matrice minérale.

9. Utilisation selon la revendication 8, **caractérisée en ce que** R₁ représente un groupe alkyle supérieur qui compte de préférence 12, 14, 16 ou 18 atomes de carbone, R₂, R₃ et R₄ représentant chacun un groupe méthyle et Y représentant l'azote.

10. Utilisation selon la revendication 8, **caractérisée en ce que** R₁ représente un groupe aralkyle, de préférence un groupe benzyle, R₂ représente un groupe alkyle supérieur qui compte de préférence 12, 14 ou 16 atomes de carbone et R₃ et R₄ représentent chacun un groupe méthyle.

11. Utilisation selon l'une des revendications 8 à 10, **caractérisée en ce que** le contre-anion X représente un bromure, chlorure, phosphate, sulfate, formiate, acétate, propionate, oxalate ou succinate.

12. Utilisation selon l'une des revendications 8 à 11, **caractérisée en ce que** la matrice minérale est constituée d'un support poreux et/ou non poreux à base d'oxydes métalliques ou d'oxydes métalliques mixtes.

13. Utilisation selon la revendication 12, **caractérisée en ce que** la matrice minérale est constituée d'un composé de silicium et d'oxygène, de préférence de dioxyde de silicium ou d'un silicate.

14. Utilisation selon la revendication 12, **caractérisée en ce que** la matrice minérale est constituée de verre, de silicagel ou d'une zéolithe.

15. Utilisation selon la revendication 12, **caractérisée en ce que** la matrice minérale est constituée d'oxyde d'aluminium, de dioxyde de titane et/ou de dioxyde de zirconium.

16. Utilisation selon l'une des revendications 13 à 15, **caractérisée en ce que** l'on utilise des mélanges desdits oxydes métalliques.

17. Utilisation selon l'une des revendications 8 à 16, pour lier des acides nucléiques qui présentent en particulier la forme d'ADN et/ou d'ARN à simple brin ou à double brin ainsi que de nucléotides et/ou de ribonucléotides individuels.

18. Utilisation selon la revendication 17, **caractérisée en ce que** l'ADN est de l'ADN génomique, de l'ADN de plasmide et/ou de l'ADN plastidaire.

19. Utilisation selon la revendication 17, **caractérisée en ce que** l'ARN est de l'ARNm, de l'ARNt, de l'ARNr et/ou de l'ARNsn.
